# EUROPEAN PATENT APPLICATION

(11) **EP 4 060 527 A1**
(43) Date of publication of application: **21.09.2022**
(21) Application number: 22162342.4
(22) Date of filing: 15.03.2022
(51) Int. Cl.: G06F 21/32, B60K 28/06, G06F 21/34, G06N 3/04

(54) **METHOD FOR REMOTE CONTROL OF ALCOHOL CONTENT IN EXHALED AIR**

(30) Priority: 15.03.2021 PL 43729621
(71) Applicant: Aidlermedia Spolka Z Ograniczona Odpowiedzialnoscia, 02-495 Warszawa (PL)
(72) Inventor: RAKIVNENKO, Vasyl, 02-495 Warszawa (PL)
(74) Representative: Patpol Kancelaria Patentowa Sp. z o.o.

(57) **Abstract**

The object of the invention is a method for remotely checking alcohol content in exhaled air, applicable for conditioning access to workstations by means of a computer application. The creation of a suitable model allows development of a virtual assistant to support the work of the sobriety supervisor at the workstation. A method for remotely checking alcohol content in exhaled air, wherein alcohol concentration in exhaled air is measured using a checking device (UK), by default a breathalyser with radio transmission technology, is characterised in that a created set of reference data (DW) comprising individualising electronic traces of the checking device (UK) and a user (U) is stored in a web application data acquisition module (MGAW) on a server contained within a data analysis network infrastructure (ISAD), the said data are then used to train a deep convolutional neural network model the task of which is to classify and identify characteristic visual personal facial features of the users (U), individual characteristics that identify the checking device (UK) such as dimensions, and individual QR code or barcode, wherein this information is compared with the value of alcohol concentration in exhaled air, obtained from the checking device (UK), associated in a mobile device (UM) with the date, time, geographical location, and with the said predefined QR code or individualising barcode which are the electronic trace of the checking device (UK) and with the visual characteristics of the user (U) obtained from a camera of said mobile device (UM), additionally with fingerprints, an individual personalisation code of the user (U) entered manually into the mobile device (UM), wherein the information obtained being then digitised and saved in any format via telecommunication links on the above-mentioned server contained within the data analysis network infrastructure (ISAD) in the web application data acquisition module (MGAW) for comparison with reference information, wherein by means of telecommunications links, push or SMS notifications about the need to perform a test are sent from the said server to the mobile device (UM) at any time intervals, preferably at the same time of the day and time, obliging the user (U) to perform measurement activities under the condition that access to the workstation is allowed or denied, , wherein
• if the result of measurement of alcohol concentration in the air exhaled by the user (U) measured by the checking device (UK) is within tolerance limits set in the (MGAW), then the data analysis network infrastructure (ISAD) server sends an information unblocking the access to the workstation,
• if the result of measurement of alcohol concentration in the air exhaled by the user (U) measured by the checking device (UK) is above the tolerance limits set in the web application data acquisition block (MGAW), then the data analysis network infrastructure (ISAD) server sends an information blocking the access to the workstation,
• if the data analysis network infrastructure (ISAD) server does not receive feedback on alcohol concentration in the air exhaled by the user (UK) from the checking device (UK) via the mobile device (UM) within the set time period, or receives incomplete information including only the result of measurement of alcohol concentration in exhaled air with incomplete individualising electronic trace of the checking device (UK) and the user (U), then the server sends the information blocking the access to the workstation,
• if the result of measurement of alcohol concentration in the air exhaled by the user (U) measured by the checking device (UK) is consistent with or is above the tolerance set in the web application data acquisition block (MGAW) and the electronic trace of the checking device that identifies the device or the user is inconsistent, then the server sends the information blocking the access to the workstation.

## Description

The object of the invention is a method for remotely checking alcohol content in exhaled air, applicable for conditioning access to workstations by means of a computer application. The creation of a suitable model allows development of a virtual assistant to support the work of the sobriety supervisor at the workstation.

There are already known methods for remotely supervising and checking sobriety of employees, involving remote measurement of alcohol content in exhaled air and reporting the result to the employer based on telecommunications systems, with the possibility of remotely blocking the access to the workstation. Known solutions do not include formulas for detecting forgery as well as mistakes in the identification of the person subject to the measurement, with the possibility of self-learning.

In the solution according to the invention, a set of operations has been developed, including data preparation, data transformation and analysis which will allow an optimal model for device and user identification to be created.

From the Canadian patent document CA2906116, a method is known for monitoring sobriety by means of a hand-held breath-testing device which, upon receiving an alcohol concentration value in exhaled air, generates a signal containing data on the content of this substance and the user's identification data and transmits it wirelessly via a cellular network to a receiving station.

In turn, from the US invention US2020191769A1, a method is known for remotely monitoring sobriety by means of wireless devices connected via a communication network, providing users with various forms of alerts in the form of electronic messages.

From yet another US patent application US2020256848, a system is known for monitoring sobriety of the user. This system may comprise a testing device that generates a signal of the substance content. The testing device may further comprise a mouthpiece and a user identification device. The user identification device may generate the user's identification data in response to the user's breath and may transmit it from the testing device to a monitoring station. The testing device may further comprise at least one LCD screen or a light-emitting diode (LED). At least one of the LCD screens or LEDs may display at least one randomly-generated visible identification mark.

And also from the US patent application US2020388117, a method is known for monitoring sobriety by means of a hand-held breath-testing device which, upon receiving the user's breath, generates a signal containing substance content data and user identification data and wirelessly transmits the signal to a receiving station, wherein the breath testing device comprises a fingerprint reader.

The aim of the invention is to develop a method for measuring alcohol content in exhaled air of users, eliminating the possibility of false and mistaken measurement as to the device and the user.

Solving the automatic identification problem according to the proposed method allows for continuous evaluation of the recognition model.

A method for remotely checking alcohol content in exhaled air, wherein the alcohol concentration in exhaled air is measured using a checking device (UK), by default a breathalyser with radio transmission technology, is characterised in that a created set of reference data (DW) comprising the individualising electronic traces of the checking device (UK) and of a user (U) is stored in a web application data acquisition module (MGAW) on a server contained within a data analysis network infrastructure (ISAD), which are then used to train a deep convolutional neural network model the task of which is to classify and identify characteristic visual personal facial features of the users (U), individual identifying characteristics of the checking device (UK) such as dimensions and individual QR code or barcode, wherein this information is compared with the value of alcohol concentration in exhaled air obtained from the checking device (UK), associated in a mobile device (UM) with the date, time, geographical location, and with the said predefined QR code or individualising barcode which is the electronic trace of the checking device (UK) and with the visual characteristics of the user (U) obtained from a camera of the said mobile device (UM), in addition with fingerprints, an individual personalisation code of the user (U) entered manually into the mobile device (UM), the information obtained is then digitised and saved in any format via telecommunication links on the above-mentioned server contained within the data analysis network infrastructure (ISAD) in the web application data acquisition module (MGAW) for comparison with reference information, wherein by means of telecommunications links, push or SMS notifications about the need to perform a test are sent from the said server to the mobile device (UM) at any time intervals, preferably at the same time of the day and time, obliging the user (U) to perform measurement activities under the condition that access to the workstation is allowed or denied, wherein
- if the result of the measurement of alcohol concentration in the air exhaled by the user (U) measured by the checking device (UK) is within tolerance limits set in the (BGAW), then the data analysis network infrastructure (ISAD) server sends an information unblocking the access to the workstation,
- if the result of the measurement of alcohol concentration in the air exhaled by the user (U) measured by the checking device (UK) is above the tolerance limits set in the web application data acquisition block (BGAW), then the data analysis network infrastructure (ISAD) server sends an information blocking the access to the workstation,
- if the data analysis network infrastructure (ISAD) server does not receive feedback on alcohol concentration in the air exhaled by the user (UK) from the checking device (UK) via the mobile device (UM) within the set time period, or receives incomplete information including only the result of measurement of alcohol concentration in exhaled air with incomplete individualising electronic trace of the checking device (UK) and the user (U), then the server sends the information blocking the access to the workstation,
- if the result of the measurement of alcohol concentration in the air exhaled by the user (U) measured by the checking device (UK) is consistent with or is above the tolerance set in the web application data acquisition block (BGAW) and the electronic trace of the checking device that identifies the device or the user is inconsistent, then the server sends the information blocking the access to the workstation.

Preferably, individual user (U) accounts are created in the web application data acquisition module (BGAW), where historical data is stored, including the date, the individual trace of the device (UK) and the user (U), the result of the measured alcohol concentration in the blood.

Preferably, in the web application data acquisition module (BGAW), a measurement time interval is set and includes time of the day, date, time, or time between preset measurement times, or randomness of measurement in any time period, with the limit of tolerance of alcohol concentration in exhaled air.

Preferably, the workstation is a means of transport or an element of production infrastructure, in particular a car, a production machine, a warehouse truck, an aircraft, a production machine, or access gates, with the server being connected to an electronic ignition or access control device.

Preferably, the deep convolutional neural network model for recognising the user's (U) face, that is part of the data analysis network infrastructure (ISAD), is trained continuously, which means that when new data appears in the reference data (DW) storage block, in the next training iteration the neural network model is trained using more examples.

Preferably, in the web application data acquisition module (BGAW), a reference image of the user's (U) face is created on the basis of image files by default acquired from the camera of the mobile device (UM) and compared at time intervals with the image acquired within the individualising electronic trace of the checking device (UK) and the user (U), conditioning, with its consistency, the access to the workstation of said user (U), wherein the consistency is determined by the percentage of characteristic visual elements of the user's (U) face, which is stored in the reference data (DW) storage module, the said data can then be used to train the deep convolutional neural network model the task of which is to determine in percentage intervals the characteristic features of the user's (U) face.

Preferably, e-mail addresses or telephone numbers are added to the web application data collection module (BGAW), which will be notified, if the result of measurement of alcohol concentration in exhaled air is above the tolerance set in the web application data collection module (BGAW), or if in a predetermined time period, there is no feedback or the information obtained within the individualising electronic trace of the checking device (UK) and the user (U) is incomplete or inconsistent with the reference information, in particular determined with the percentage of the characteristic visual elements of the user's (U) face is below the assumed value.

Preferably, the percentage of characteristic visual elements of the user's (U) face is determined within the biometric 3D authentication of the personal characteristics of the user's (U) image.

Preferably, if the user (U) ignores the command of the data analysis network infrastructure (ISAD) server to perform the measurement of alcohol concentration in exhaled air and the positive or negative face recognition, the said server sends an email notification or a text message to the addresses defined in the web application data collection module (BGAW) in addition to blocking the workstation.

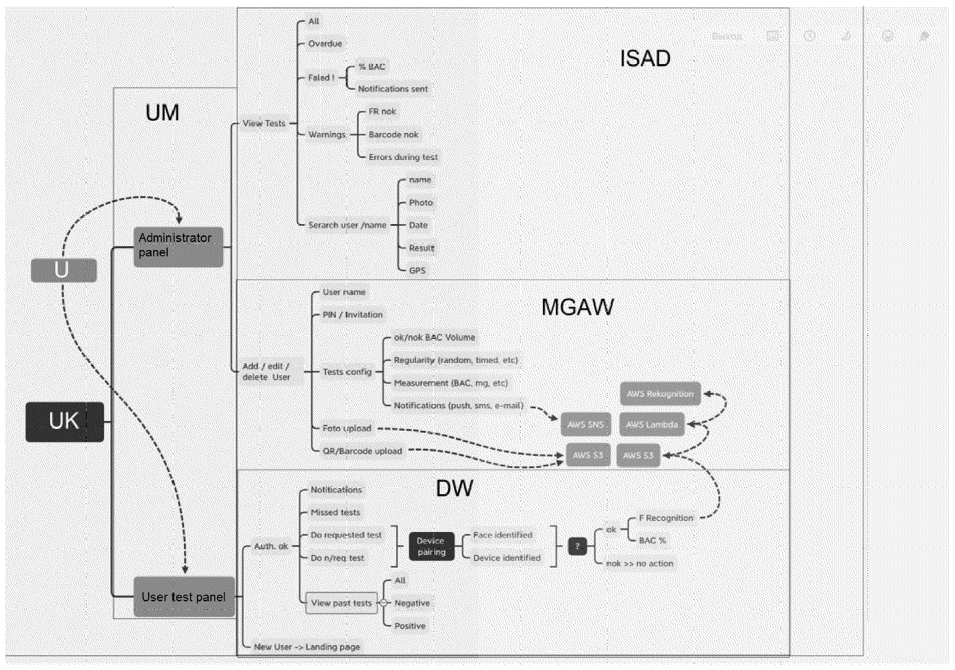

The object of the invention is presented in an embodiment in the drawing which shows a block diagram illustrating the method in question.

The use of the invention allows very precise correlation of the user, the measuring device and the result of measurement of alcohol concentration in exhaled air, with a very short time of at most several seconds. Models are 'trained' on the basis of reference data, then used to identify individual facial characteristics and individual device characteristics. The selection of models is done by using appropriate evaluation criteria and choosing the best model obtained so far. The entire optimisation process for the new models is automated and requires no human intervention.

A method for remotely checking alcohol content in exhaled air, wherein alcohol concentration in exhaled air is measured using a checking device (UK), by default a breathalyser with radio transmission technology, **characterised in that a** created set of reference data (DW) comprising individualising electronic traces of the checking device (UK) and a user (U) is stored in a web application data acquisition module (MGAW) on a server contained within a data analysis network infrastructure (ISAD), the said data are then used to train a deep convolutional neural network model the task of which is to classify and identify characteristic visual personal facial features of the users (U), individual characteristics that identify the checking device (UK) such as dimensions, and individual QR code or barcode, wherein this information is compared with the value of alcohol concentration in exhaled air, obtained from the checking device (UK), associated in a mobile device (UM) with the date, time, geographical location, and with the said predefined QR code or individualising barcode which are the electronic trace of the checking device (UK) and with the visual characteristics of the user (U) obtained from a camera of the said mobile device (UM), additionally with fingerprints, an individual personalisation code of the user (U) entered manually into the mobile device (UM), the information obtained is then digitised and saved in any format via telecommunication links on the above-mentioned server contained within the data analysis network infrastructure (ISAD) in the web application data acquisition module (MGAW) for comparison with reference information, wherein by means of telecommunications links, push or SMS notifications about the need to perform a test are sent from the said server to the mobile device (UM) at any time intervals, preferably at the same time of the day and time, obliging the user (U) to perform measurement activities under the condition that access to the workstation is allowed or denied, wherein

if the result of measurement of alcohol concentration in the air exhaled by the user (U) measured by the checking device (UK) is within tolerance limits set in the (BGAW), then the data analysis network infrastructure (ISAD) server sends an information unblocking the access to the workstation, if the result of measurement of alcohol concentration in the air exhaled by the user (U) measured by the checking device (UK) is above the tolerance limits set in the web application data acquisition block (BGAW), then the data analysis network infrastructure (ISAD) server sends an information blocking the access to the workstation,

if the data analysis network infrastructure (ISAD) server does not receive feedback on alcohol concentration in the air exhaled by the user (UK) from the checking device (UK) via the mobile device (UM) within the set time period, or receives incomplete information including only the result of measurement of alcohol concentration in the air exhaled with incomplete individualising electronic trace of the checking device (UK) and the user (U), then the server sends the information blocking access to the workstation, if the result of the measurement of alcohol concentration in the air exhaled by the user (U) measured by the checking device (UK) is consistent with or is above the tolerance set in the web application data acquisition block (BGAW) and the electronic trace of the checking device that identifies the device or the user is inconsistent, then the server sends the information blocking access to the workstation,

if the user (U) ignores a command from the data analysis network infrastructure (ISAD) server to perform a measurement of alcohol concentration in exhaled air and a positive or negative facial recognition, the said server sends an email notification or a text message to the addresses defined in the web application data acquisition module (BGAW) in addition to blocking the workstation.

In the web application data acquisition module (BGAW), individual user (U) accounts are created in which historical data is stored, including the date, the individual trace of the device (UK) and the user (U), the result of measured alcohol concentration in the blood. In the web application data acquisition module (BGAW), a measurement interval is set, including time of the day, date, hour, or time between set measurement hours, or randomness of measurement in any time period, with a tolerance limit of alcohol in exhaled air.

The workstation is a means of transport or an element of production infrastructure, in particular a car, a production machine, a warehouse truck, an aircraft, a production machine or access gates, wherein the server is connected to an electronic ignition or access control device. The deep convolutional neural network model for recognising the user's (U) face, which is part of the data analysis network infrastructure (ISAD) is trained continuously, which means that when new data appears in the reference data (DW) storage block, in the next training iteration the neural network model is trained using more examples.

In the web application data acquisition module (BGAW), a reference image of the user's (U) face is created on the basis of image files by default acquired from the camera of the mobile device (UM) and compared at time intervals with the image acquired within the individualising electronic trace of the checking device (UK) and of the user (U), conditioning, with its consistency, the access to the workstation of the said user (U), wherein this consistency is determined by the percentage of characteristic visual elements of the user's (U) face, which is stored in the reference data (DW) storage module, the said data can then be used to train the deep convolutional neural network model the task of which is to determine in percentage intervals the characteristic features of the user's (U) face.

E-mail addresses or telephone numbers are added to the web application data collection module (BGAW), which will be notified, if the result of measurement of alcohol concentration in exhaled air is above the tolerance set in the web application data collection module (BGAW), or if in a predetermined time period, there is no feedback or the information obtained within the individualising electronic trace of the checking device (UK) and the user (U) is incomplete or inconsistent with the reference information, in particular determined with the percentage of the characteristic visual elements of the user's (U) face is below the assumed value. The percentage of characteristic visual elements of the user's (U) face is determined within the biometric 3D authentication of the personal characteristics of the user's (U) image.

## Claims

1. A method for remotely checking alcohol content in exhaled air, wherein alcohol concentration in exhaled air is measured using a checking device (UK), by default a breathalyser with radio transmission technology, **characterised in that a** created set of reference data (DW) comprising individualising electronic traces of the checking device (UK) and a user (U) is stored in a web application data acquisition module (MGAW) on a server contained within a data analysis network infrastructure (ISAD), the said data are then used to train a deep convolutional neural network model the task of which is to classify and identify characteristic visual personal facial features of the users (U), individual characteristics that identify the checking device (UK) such as dimensions, and individual QR code or barcode, wherein this information is compared with the value of alcohol concentration in exhaled air, obtained from the checking device (UK), associated in a mobile device (UM) with the date, time, geographical location, and with the said predefined QR code or individualising barcode which are the electronic trace of the checking device (UK) and with the visual characteristics of the user (U) obtained from a camera of said mobile device (UM), additionally with fingerprints, an individual personalisation code of the user (U) entered manually into the mobile device (UM), wherein the information obtained being then digitised and saved in any format via telecommunication links on the above-mentioned server contained within the data analysis network infrastructure (ISAD) in the web application data acquisition module (MGAW) for comparison with reference information, wherein by means of telecommunications links, push or SMS notifications about the need to perform a test are sent from the said server to the mobile device (UM) at any time intervals, preferably at the same time of the day and time, obliging the user (U) to perform measurement activities under the condition that access to the workstation is allowed or denied, wherein
• if the result of measurement of alcohol concentration in the air exhaled by the user (U) measured by the checking device (UK) is within tolerance limits set in the (MGAW), then the data analysis network infrastructure (ISAD) server sends an information unblocking the access to the workstation,
• if the result of measurement of alcohol concentration in the air exhaled by the user (U) measured by the checking device (UK) is above the tolerance limits set in the web application data acquisition block (MGAW), then the data analysis network infrastructure (ISAD) server sends an information blocking the access to the workstation,
• if the data analysis network infrastructure (ISAD) server does not receive feedback on alcohol concentration in the air exhaled by the user (UK) from the checking device (UK) via the mobile device (UM) within the set time period, or receives incomplete information including only the result of measurement of alcohol concentration in exhaled air with incomplete individualising electronic trace of the checking device (UK) and the user (U), then the server sends the information blocking the access to the workstation,
• if the result of measurement of alcohol concentration in the air exhaled by the user (U) measured by the checking device (UK) is consistent with or is above the tolerance set in the web application data acquisition block (MGAW) and the electronic trace of the checking device that identifies the device or the user is inconsistent, then the server sends the information blocking the access to the workstation.

2. The method for remotely checking alcohol content of users according to claim 1 **characterised in that** individual user (U) accounts are created in the web application data acquisition module (MGAW), where historical data is stored, including the date, the individual trace of the device (UK) and the user (U), the result of the measured alcohol concentration in the blood.

3. The method for remotely checking alcohol content of users according to claim 1 **characterised in that** in the web application data acquisition module (MGAW), a measurement time interval is set and includes time of the day, date, time, or time between preset measurement times, or randomness of measurement in any time period, with the limit of tolerance of alcohol concentration in exhaled air.

4. The method for remotely checking alcohol content of users according to claim 1 **characterised in that** the workstation is a means of transport or an element of production infrastructure, in particular a car, a production machine, a warehouse truck, an aircraft, a production machine, or access gates, with the server being connected to an electronic ignition or access control device.

5. The method for remotely checking alcohol content of users according to claim 1 **characterised in that** the deep convolutional neural network model for recognising the user's (U) face, that is part of the data analysis network infrastructure (ISAD) is trained continuously, which means that when new data appears in the reference data (DW) storage block, in the next training iteration the neural network model is trained using more examples.

6. The method for remotely checking alcohol content of users according to claim 1 **characterised in that** in the web application data acquisition module (MGAW), a reference image of the user's (U) face is created on the basis of image files by default acquired from the camera of the mobile device (UM) and compared at time intervals with the image acquired within the individualising electronic trace of the checking device (UK) and the user (U), conditioning, with its consistency, the access to the workstation of said user (U), wherein the consistency is determined by the percentage of characteristic visual elements of the user's (U) face, which is stored in the reference data (DW) storage module, the said data can then be used to train the deep convolutional neural network model the task of which is to determine in percentage intervals the characteristic features of the user's (U) face.

7. The method for remotely checking alcohol content of users according to claim 1 **characterised in that** e-mail addresses or telephone numbers are added to the web application data collection module (MGAW), which will be notified, if the result of measurement of alcohol concentration in exhaled air is above the tolerance set in the web application data collection module (MGAW), or if in a predetermined time period, there is no feedback or the information obtained within the individualising electronic trace of the checking device (UK) and the user (U) is incomplete or inconsistent with the reference information, in particular determined with the percentage of the characteristic visual elements of the user's (U) face is below the assumed value.

8. The method for remotely checking alcohol content of users according to claim 5 or 6, **characterised in that** the percentage of characteristic visual elements of the user's (U) face is determined within a biometric 3D authentication of the personal characteristics of the user's (U) image.

9. The method for remotely checking alcohol content of users according to claim 1, **characterised in that** if the user (U) ignores a command of the data analysis network infrastructure (ISAD) server to perform the measurement of alcohol concentration in exhaled air and the positive or negative face recognition, the said server sends an email notification or a text message to the addresses defined in the web application data collection module (MGAW) in addition to blocking the workstation.
